# EUROPEAN PATENT SPECIFICATION

(11) **EP 2 321 311 B1**
(45) Date of publication and mention of the grant of the patent: **19.09.2012**
(21) Application number: 09777171.1
(22) Date of filing: 10.07.2009
(51) Int. Cl.: C07D 471/04

(54) **SYNTHESIS OF PALIPERIDONE**
SYNTHESE VON PALIPERIDON
SYNTHÈSE DE PALIPÉRIDONE

(30) Priority: 11.07.2008 US 80072 P
(43) Date of publication of application: 18.05.2011
(73) Proprietor: Synthon B.V., 6545 CM Nijmegen (NL)
(72) Inventor: BARTL, Jiri, 664 47 Strelice (CZ); BENOVSKY, Petr, 614 00 Brno (CZ)
(74) Representative: Prins, Hendrik Willem
(86) International application number: PCT/EP2009/005099
(87) International publication number: WO 2010/003702

(56) References cited:
- EP-A- 0 368 388
- WO-A-2009/074333
- FANG ET AL: "Metabolismof risperidone to 9-hydroxyrisperidone by human cytochromes P450 2D6 and 3A4" NAUNYN-SCHMIEDEBERG'S ARCHIVES OF PHARMACOLOGY, SPRINGER, BERLIN, DE, vol. 359, 1 January 1999 (1999-01-01), pages 147-151, XP002494407 ISSN: 1432-1912
- YASUI-FURUKORI ET AL: "Different enantioselective 9-hydroxylation of risperidone by the two human CYP2D& and CYP3A4 enzymes" DRUG METABOLISM AND DISPOSITION, WILLIAMS AND WILKINS, BALTIMORE, MD, US, vol. 29, no. 9, 1 January 2001 (2001-01-01), pages 1263-1268, XP002494408 ISSN: 0090-9556

## Description

### BACKGROUND OF THE INVENTION

Paliperidone, or 9-hydroxyrisperidone (chemically: (±)-3-(2-(4-(6-fluoro-1,2-benzisoxazol-3-yl)-1-piperidinyl)ethyl)-6,7,8,9-tetrahydro-9-hydroxy-2-methyl-4H-pyrido[1,2-a]pyrimidin-4-one) of the formula (I) 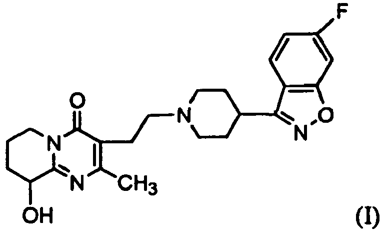 is a major human metabolite of the known antipsychotic drug risperidone. It is marketed in tablets for oral administration under the brand name INVEGA™ (Janssen, L.P.) for treatment of schizophrenia. Paliperidone has one centre of optical activity (the carbon in the 9-position); both enantiomers are known but the marketed compound is a racemate.

Paliperidone (including enantiomeric forms thereof) has been disclosed in EP 368388 (US 5158952). EP 368388 also discloses esters of paliperidone with carboxylic acids having the formula (II) (R'= C₁₋₁₉ alkyl).
Fang, Navayn-Schmiedeberg's Archives of Pharmacology, vol 359, 1999, 197-156 and Yasin-Forokori, Drug Metabolism and Disposition, vol 29(9), 2001, 1263-1268 disclose the production of paliperidone from risperidone via enzymatic hydroxylation.

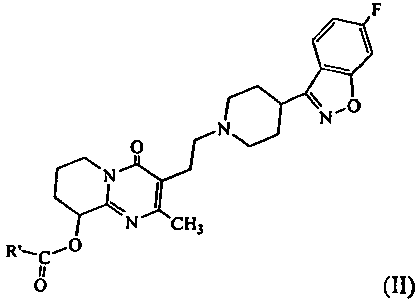

A preferred ester compound (II) is paliperidone palmitate, which is currently under development for use in injectable compositions with prolonged action.

Various processes for making compounds of formula (I) and (II) have been generally disclosed in the EP 368388. One process is based on an alkylation of a 3-piperidinyl 1,2-benzisoxazole of the formula (2) with the compound of formula (1.1), wherein R is hydrogen or C1-C20 acyl group and A represents an appropriate leaving group such as, for example, halo, e.g., chloro, bromo or iodo; sulfonyloxy, e.g., methanesulfonyloxy, trifluoromethanesulfonyloxy, benzenesulfonyloxy, 4-methylbenzenesulfonyloxy and the like leaving groups.

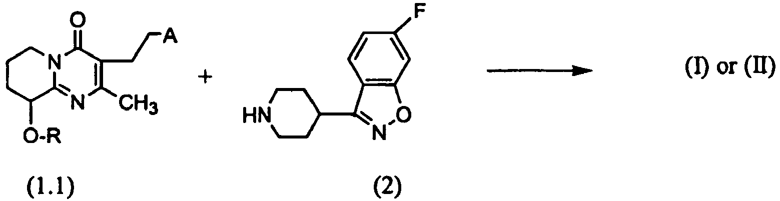

Another known process is based on the reaction of the compound (1.1) with an oxime compound (3), wherein L is a reactive leaving group, followed by the ring closure of the isoxazole ring on the intermediate (4):

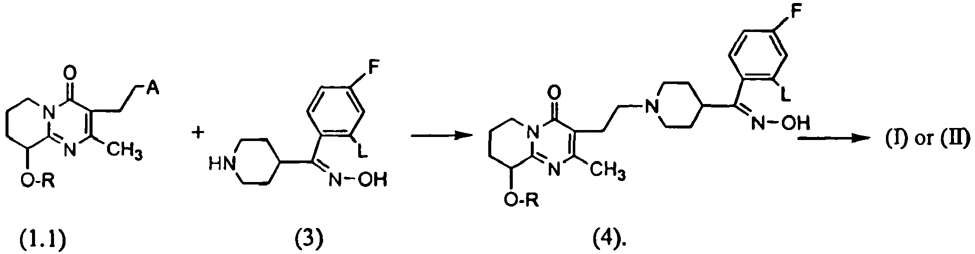

Furthermore, the esters of formula (II) may be prepared by acylating paliperidone (I) by an acylation agent (e.g. acyl halide or acyl anhydride).

The compounds of the general formula (1.1) are valuable intermediates in making paliperidone (I) as well as the paliperidone esters of the formula (II). A typical example of the intermediate of the general formula (1.1) is the compound 3-(2-chloroethyl)-6,7,8,9-tetrahydro-9-hydroxy-2-methyl-4H-pyrido[1,2-a]pyrimidin-4-one of the formula (1a)

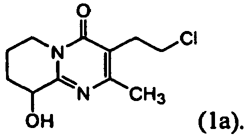

A known process for making the compounds of general formula (1.1) is based on making the corresponding O-protected starting material, followed by a reductive deprotection. An example of such process for making (1a) is shown below in Scheme I (Bn is benzyl group)

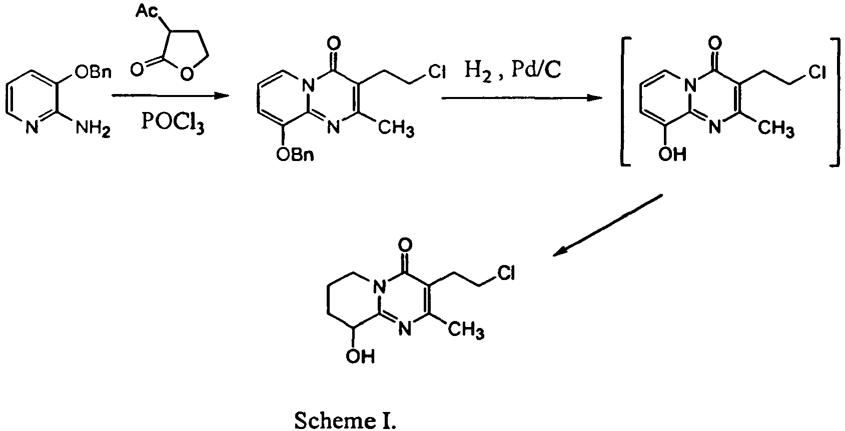

The yield in such a process is generally low, e.g. 50%, and the compound (1a) is not readily obtained as a crystalline compound under the usual reaction conditions, which is also disadvantageous.

As paliperidone is structurally very similar to the known antipsychotic drug risperidone, the synthesis of which is well known and elaborated, it would be convenient to use risperidone or its starting materials in the synthesis of paliperidone and its O-acyl analogues. For instance, the risperidone starting materials of formula (5.1), which lacks the 9-OH group of formula (1) that is needed in the final paliperidone, are commercially available (A=Cl) and can be produced in good yields. 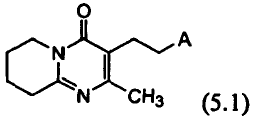 But, no process for a conversion of the compound (5.1) (or its subsequent intermediates through risperidone) into the compound (1.1) (and the corresponding subsequent intermediates through paliperidone) was found to have been disclosed in the art.

### SUMMARY OF THE INVENTION

The present invention relates to the discovery of a synthetic pathway for providing a 9-hydroxy group on risperidone or its intermediates/starting materials and is thus useful in making paliperidone, its derivatives, intermediates, and/or starting materials.

A first aspect of the present invention is directed to a compound of formula (6) or an acid addition salt thereof 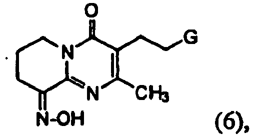 wherein G is selected from the group consisting of:
(i) a leaving group A selected from a halo group and a sulfonyloxy group, and
(ii) a group of the formula (20)

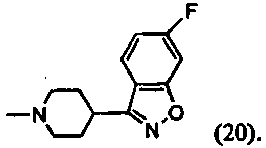

Typically, when G is the leaving group A, G is selected from chloro, bromo, iodo, methanesulfonyloxy, trifluoromethanesulfonyloxy, benzenesulfonyloxy, 4-methylbenzenesulfonyloxy, and 4-methoxybenzenesulfonyloxy. In a preferred embodiment, G is chlorine.

A second aspect of the present invention is directed to a process, which comprises:
a) reacting a compound of formula (5) or a salt thereof 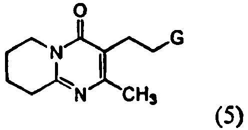 with a nitrite reagent to form a compound of formula (6) or a salt thereof 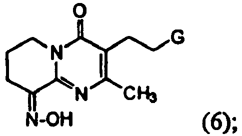
b) deoximating the compound of formula (6) or salt thereof to form a compound of formula (7) or a salt thereof 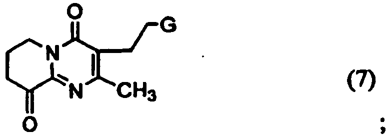 and
c) reducing the compound of formula (7) or salt thereof to form a compound of formula (1) or a salt thereof 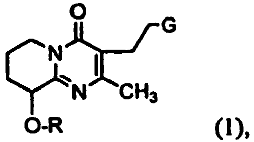 wherein G in each of the formulas (5), (6), (7), and (1) is selected from the group consisting of:
   (i) a leaving group A selected from a halo group and a sulfonyloxy group, and
   (ii) a group of the formula (20) 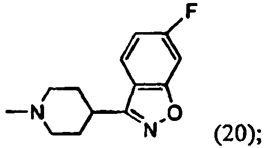 and
   wherein R in the compound of formula (1) is hydrogen or a C1-C20 acyl group.
   When G in the compound of formula (5) is the leaving group A, the process further comprises converting the leaving group A on any one of the subsequent compounds of formulas (6), (7), and (1) into the group of the formula (20). Additionally, when R in the compound of formula (1) is hydrogen, the process further comprises converting the hydrogen into a C1-C20 acyl group. The compound of formula (6) formed in step a) and the compound of formula (1) formed in step c) can be racemates, single enantiomers, or non-racemic mixtures of enantiomers.

### DETAILED DESCRIPTION OF THE INVENTION

The present invention relates to the discovery of a new process for adding a 9-hydroxy group to a tetrahydropyridopyrimidin-4-one ring system. By use of the invention, the knowledge of the synthesis of risperidone can be easily used to produce paliperidone or the esters thereof. The process can be represented by the following general scheme. 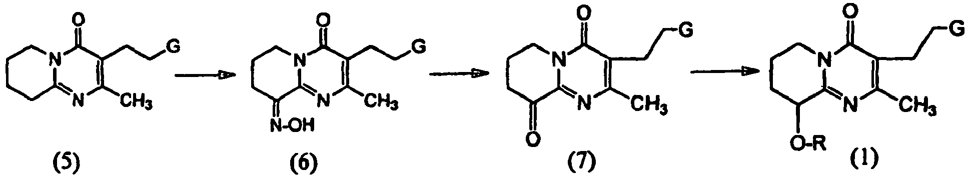 and its esters is shown below. In this diagram the double reaction arrow represents the oximation, deoximation and reduction steps of the present invention; and (A), (B), (C), (D) denote four principal variants of the inventive process disclosed in more detail below:

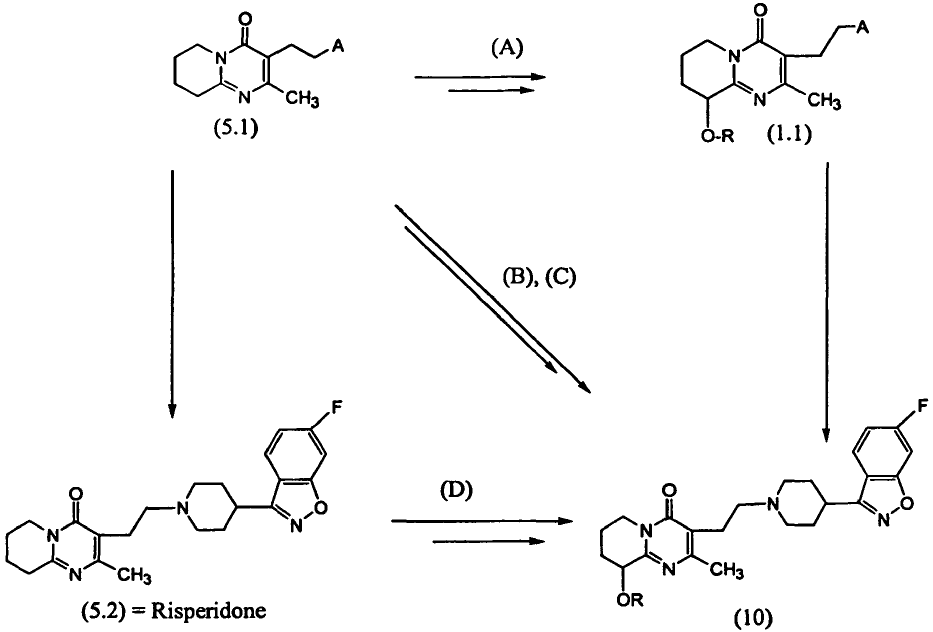

The left-most vertical arrow reflects any conventional route for making risperidone starting from a compound of formula (5) where G represents a leaving group "A" and denoted as compound (5.1). Subjecting a compound of (5.1) to the steps of oximation, deoximation, and reduction as per the present invention [variant (A)] results in the classic paliperidone starting material, denoted as (1.1), e.g. a compound of formula (1) wherein G represents a leaving group "A" and R represents hydrogen. The compound of formula (1.1) may be then converted into paliperidone conventionally (the right-most vertical arrow). Such conversion generally comprises a reaction of the compound (1.1) with 4-(6-fluoro-1,2-benzisoxazol-3-yl)piperidine (compound (2)) or with a precursor thereof, which may be a oxime compound (3) or a keto-compound (3A), as exemplified on the scheme below, which employs the compound (1a) (i.e., the compound of formula (1.1) wherein A = Cl and R = H) as a suitable starting material: 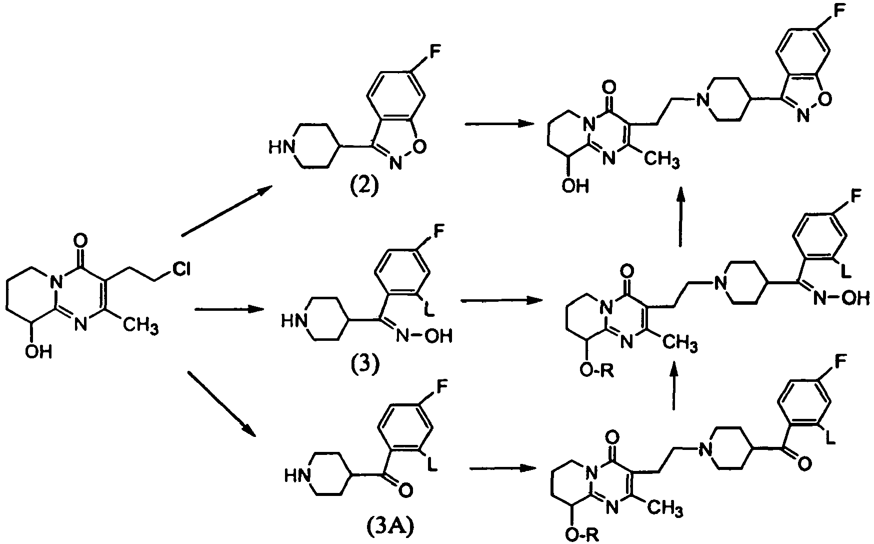 If a product of the oximation reaction or the deoximation reaction is contacted with the 4-(6-fluoro-1,2-benzisoxazol-3-yl) piperidine prior to the reduction step, then the paliperidone may be made by variants (B) and (C), respectively, as discussed in more detail below. Furthermore, the oximation, deoximation, and reduction steps of the present invention can be carried out on risperidone itself, e.g. using a compound of formula (5.2) as the starting material, i.e., the compound of formula (5) wherein A = the group of formula (20) 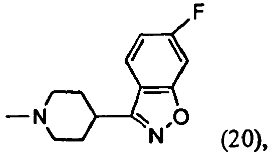 resulting in a compound of formula (10), e.g. paliperidone (R = H) or its esters (variant (D)).

For clarity, unless specifically stated otherwise, all formulas and compounds mentioned herein include the salts thereof, especially pharmaceutically acceptable salts, as well as the individual isomers and mixtures of isomers including racemic mixtures. Further, a "step" may include multiple reactions, conditions, and/or reagents, which can be added at one or more times, and is not limited to a single event. The two or more "steps" of the invention may also proceed in a single technological process, e.g., two "steps" can be performed concurrently using the same reagents / reaction conditions. Thus, the "three step" process of the invention is characterized by the three general steps of oximation, deoximation, and reduction, as prescribed herein, and is not limited to three manipulations or specific events.

The first variant (A) of the process of the present invention represents a conversion of compounds of formula (5) wherein "G" represents a leaving group "A." The reaction can be represented as follows: 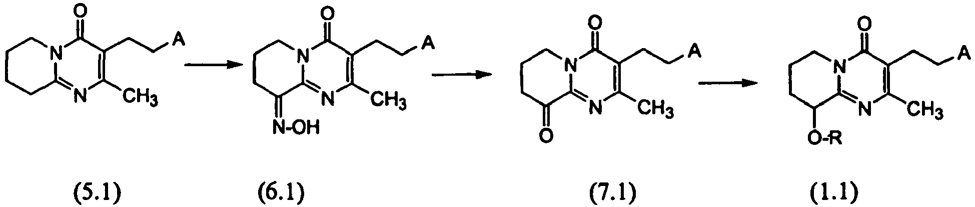 wherein R is hydrogen and A is a leaving group selected from halo( e.g., chloro, bromo, and iodo groups) and sulfonyloxy (e.g., methanesulfonyloxy, trifluoromethanesulfonyloxy, benzenesulfonyloxy, 4-methylbenzenesulfonyloxy, and 4-methoxybenzenesulfonyloxy groups). In a preferred embodiment of the above process, the compound of the general formula (5) is the compound of formula (5a), 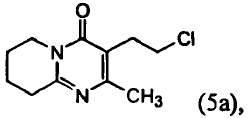 the compounds of the general formula (6) and (7) are the compounds of formula (6a) and (7a), respectively, 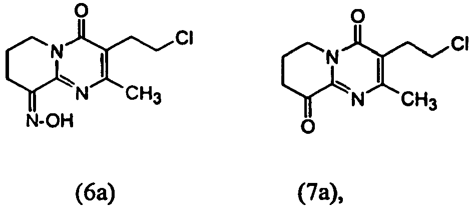 and the compound of the general formula (1) is the compound of formula (1a)

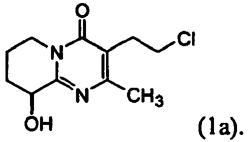

The starting material of the formula (5a) is known in the art and is commercially available. In general, it may be obtained from 2- amino pyrimidine by a similar process as shown above in the Scheme 1 and illustrated below in Scheme II:

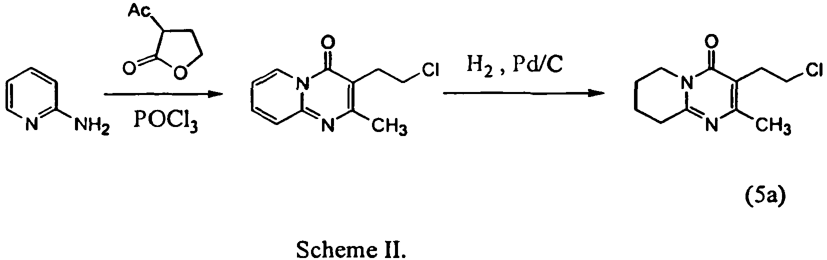

The process of Scheme II provides higher conversion than the process of Scheme I; Scheme I provides the compound (1a) generally with about 50% yield only. It is believed that Scheme I has the lower yield because the presence of the 9-OH group causes the formation of side-products. Furthermore, the compound (1a) cannot be prepared in a crystalline form by the known process, contrary to the compound (5a). Thus, it can be advantageous to build up the pyrido-pyrimidine ring according to the process of the Scheme II rather than according to the process of the Scheme I, and to use the compound (5a) or more generally (5.1) in making the desired paliperidone product. The other compounds of the general formula (5.1), e.g., compounds where A is other than Cl, may be produced similarly or may be made from the corresponding chlorine compound by the replacement of the chlorine group with an alternative A-group using methods generally known in the art.

In the first step, the compound of the general formula (5.1), preferably of the formula (5a), is converted into the compound of the general formula (6.1), preferably (6a), by an oximation reaction. The reaction proceeds by contacting, under reactive conditions, the compound of the general formula (5.1) with a "nitrite reagent." The "nitrite reagent" may be nitrous acid HNO₂ or a salt thereof with an alkali metal or an alkaline earth metal (such as sodium nitrite), an alkylnitrite R-NO₂ wherein R is an alkyl group of 1-10 carbon atoms, preferably 4-9 carbon atoms (e.g., amyl nitrite or iso-amyl nitrite), or a nitrosyl compound (e.g., as nitrosylchloride). The oximation reaction suitably proceeds in an inert solvent, which may be, e.g., water, a lower aliphatic (C1-C4) alcohol such as methanol or ethanol, or mixtures thereof. If an alkali or alkali metal nitrite is used as the nitrite reagent, then the reaction advantageously proceeds in the presence of an acid, e.g. hydrochloric acid, preferably in water. The reaction temperatures are preferably 50 - 90 °C.

The oxime reaction product (6.1) may exist in two conformations, (E) and (Z), as generally known for oximes. It may be isolated from the reaction mixture as a mixture of both of the conformers, or manipulated in such a way that the content of one conformer in the isolated product exceeds or even substantially exceeds the content of the other enantiomer. For instance, the oxime may be isolated in a form of a suitable salt with an appropriate organic or inorganic acid, and in some cases the differences in solubilities of the salts with the (E) and (Z) conformers permits one of the conformers to preferentially precipitate while the other substantially remains in solution.

Both conformers are equally suitable for the next reaction step, thus the isolation of the compound (6.1) enriched by one of the conformers is not strictly necessary. On the other hand, it is sometimes advantageous to isolate the compound (6.1) as an acid addition salt because of good handling properties. Suitable acid addition salts of the compound (6.1) include a hydrochloride salt thereof.

In general, the oximation reaction runs under mild conditions, with a high yield, providing a product with high purity. The process may yield the compound (6.1), and particularly the compound (6a), in a chemical purity higher than 95% and even higher than 99%, which is very useful in respect to the purity of the product in the next step of the process of the present invention.

In the second step, the oxime (6.1), a conformer thereof and/or an acid addition salt thereof, is converted into the keto-compound (7.1), preferably of the formula (7a), by a deoximation reaction, i.e. contacting the compound (6.1), preferably (6a), under reactive conditions with a deoximation agent in a solvent.

Deoximation is the conversion of an oxime to the corresponding carbonyl compound. The deoximation may proceed, e.g., by:
a) Oxime exchange, i.e. by the exchange of the oxime group >N-OH with a carbonyl group >C=O, e.g. by a reacting with a ketone, e.g. with acetone
b) Nucleophilic addition to the oxime group followed by a hydrolysis. This may be carried out e.g. by a bisulphite ion in the presence of water, or by formaldehyde in the presence of an acid, usually mineral acid, e.g. HCl
c) Reductive deoximation, e.g. by titanium trichloride or chromous acetate, or by a hydrogenation in the presence of a selectively poisoned catalyst.
d) Oxidative deoximation, e.g. by thallium (III) nitrate, ammonium persulfate, alkali (e.g. sodium) hypochlorite or nitrous acid in water.
e) Hydrolysis, which is the reaction with water.

This is not a rigid classification. For instance, the use of aqueous formaldehyde and acid can be regarded as the oxime exchange (a), nucleophilic addition followed by hydrolysis (b), or the hydrolysis (e). A particular deoximation process may involve elements of more than one of these classes.

Whenever made, the keto-compound (7.1), preferably the compound (7a), is converted into the compound (1.1) [R = H], preferably (1a), by a reduction process. The reduction may be made using a reductant, such as a hydride (e.g., sodium borohydride), gaseous hydrogen in the presence of a hydrogen catalyst including heterogeneous and homogenous catalysts (e.g., palladium, platinum, platinum oxide, and Raney-Nickel), and native hydrogen formed by contacting a transition metal (e.g., zinc) with an acid. The reduction may produce the compound (1.1) [R = H] as a mixture of enantiomers (a non-selective reduction), or enhanced by a single enantiomer (stereoselective reduction). In a stereoselective reduction, a chiral reductant is preferably used, such as a complex of a transition metal (e.g., ruthenium and rhodium) with one or more chiral ligands. The reduction process proceeds in a solvent system, which may be water or an organic solvent, or a mixture of both. The compound (1.1.) [R = H] is then isolated from the reaction mixture by conventional means, e.g. by a filtration.

The preferred deoximation process in the present invention is the process of the reductive deoximation. In the reductive deoximation, the formed keto-compound (7.1) is a temporarily formed intermediate, which is converted in situ into the desired final product (1.1) [R = H] without a need of isolating the ketone (7.1). Thus, the use of a single deoximation/reduction agent and one set of reactions conditions can be used to perform the deoximation and reduction steps concurrently. The preferred deoximation/reduction agent is titanium trichloride. The deoximation/reduction process advantageously proceeds in an inert solvent, e.g. on water, optionally under presence of an acid. The product (1.1.) [R = H] is then isolated by conventional means, e.g. by filtration or by an extraction, from the reaction mixture.

The compound (1.1) [R=H] may be O-acylated by corresponding acylhalide or acylanhydride into compound (1.1) [R = C1-C20 acyl].

The product of general formula (1.1) [R = H or C1-C20 acyl] may then serve as a starting material for the synthesis of paliperidone as well as derivatives thereof, including pharmaceutically acceptable salts and the stereochemically isomeric forms thereof. The conversion of the compounds of formula (1.1) into paliperidone or its esters is generally well known as shown above. The compound of formula (1.1) can be alkylated with a compound of formula (2) and/or a precursor thereof to form a compound of formula (10): 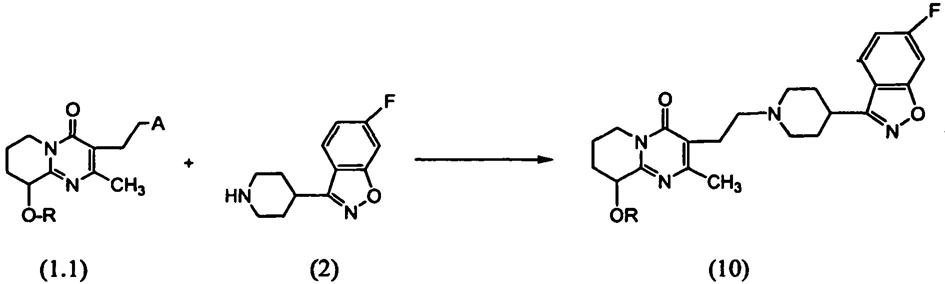 When R in formula (10) is hydrogen, the compound is paliperidone. Likewise the conversion of (1.1) to (10) can comprise alkylating with a compound of formula (3) to form a compound of formula (4) followed by ring closure, as described above. The conditions of the conversion reactions are well known in the art. Additionally, the compound (1.1) may serve as starting material for the synthesis of the 9-tetrahydropyranyl paliperidone derivative disclosed in U.S. provisional patent application serial No. 60/952,376, filed July 27, 2007. Similarly, compounds of the formula (1.1) can be used for making esters of paliperidone, e.g., paliperidone palmitate may be advantageously made from the compound (1b) [R = C1-C20 alkyl].

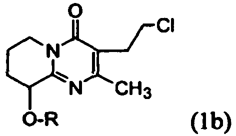

The possibility to obtain the starting compound in a high purity allows for making such paliperidone compounds with higher yields and with less burden in purification to achieve pharmaceutically acceptable quality.

The transformation of the group G from a leaving group A to the formula (2)) can occur at essentially anytime during the process. That is, the value of G is not necessarily constant from compound to compound. This is illustrated more fully below in variants (B) and (C).

In a second variant (B) of the process of the present invention, the reaction proceeds according to the following scheme:

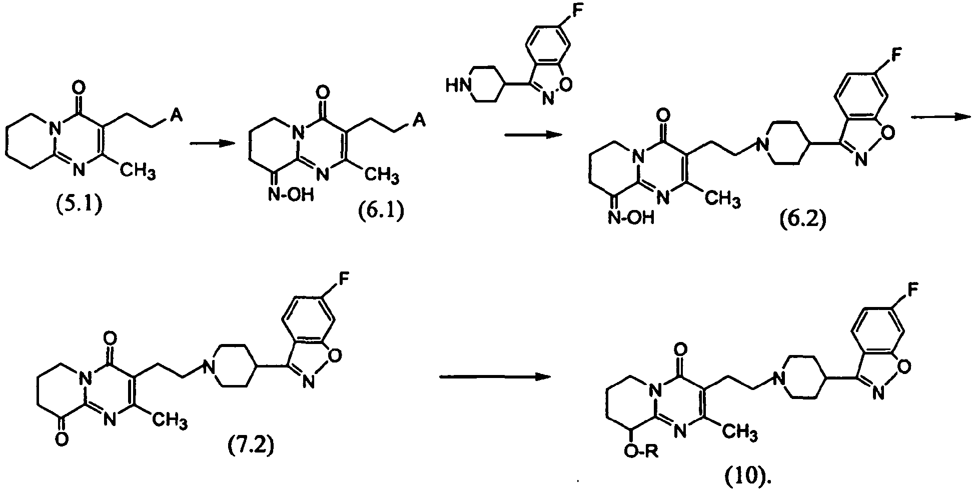

Thus, the process of variant (B) differs from the process of the variant (A) in that the compound (6.1) (or salt thereof) is converted into the compound (6.2) prior to the deoximation reaction. The formation of the compound (6.2) advantageously comprises contacting, under reactive conditions, the compound (6.1) with the compound (2) in a solvent, preferably in the presence of a base, which may be an organic or an inorganic base, e.g., a alkali or an alkaline earth metal hydroxide or carbonate (e.g., sodium hydrogen carbonate), or an amine (e.g., triethyl amine). The useful solvent is, e.g., an aliphatic alcohol, dimethylformamide, dimethyl sulfoxide and mixtures thereof.

The product (6.2) may be isolated from the reaction mixture by conventional means, e.g., by precipitation and filtration, and purified if necessary, Alternately, the product (6.2) may be subjected to the next reaction step without isolation. The product (6.2) may be prepared and/or isolated either in a free form or in a form of its acid addition, salt with a suitable acid. The choice of the acid is not specifically limited.

In the next step, the compound (6.2) is deoximated into the keto-compound of the formula (7.2), which, in turn, is reduced to yield the paliperidone (the compound (10), wherein R is hydrogen). The conditions of deoximation and reduction disclosed above for the conversion of (6.1) to (1.1) via (7.1) may be applied here as well. In particular, the deoximation and reduction may be performed in one step, e.g., by contacting, under reactive conditions, the compound (6.2) with titanium trichloride. Preferably, the process is monitored to determine whether and how much of the compound of formula (7.2) remains. The process can be terminated when the amount of the compound of formula (7.2) in the reaction mixture drops below a predetermined threshold, e.g., below 2%.

The product (7.2) may be isolated from the reaction mixture by conventional means, and purified if necessary. The product may be prepared and/or isolated either in a free form, or in a form of its acid addition salt with a suitable acid. The choice of the acid is not specifically limited.

In accordance with the teaching above, the paliperidone product may be obtained by the reduction of the keto-compound (7.2) as a racemate (the paliperidone itself, compound (I)), or enhanced by a single enantiomer, by a choice of the reduction agent.

The obtained paliperidone, including the single enantiomers thereof, may be then converted into paliperidone esters by conventional processes indicated above.

In a third variant (C) of the process of the present invention, the reaction proceeds as follows:

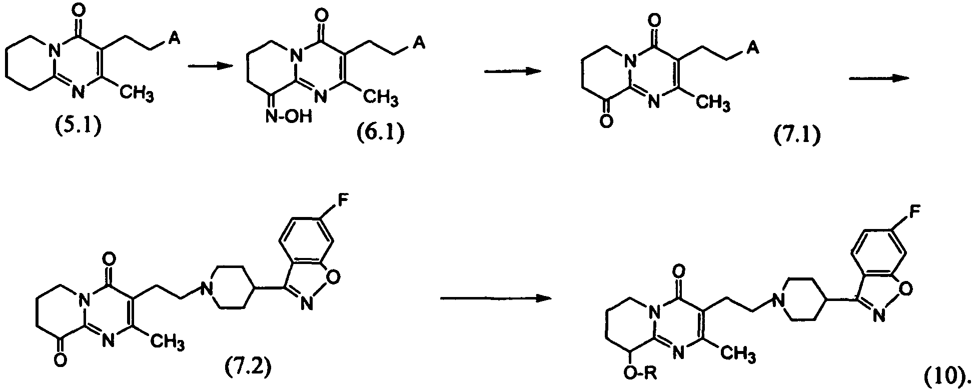

Thus, variant (C) differs from variants (A) and (B) in that the compound (7.1) is converted, preferably by reacting with the compound of formula (2), into the compound of the formula (7.2). As discussed above for the second variant (B), the process is preferably monitored to determine whether and how much of the compound of formula (7.2) is present, and the process can be terminated when the amount of the compound of formula (7.2) in the reaction mixture drops below a predetermined threshold.

The conditions of the conversion are, in general, similar to the conditions applicable for the conversion of the compound (6.1) to the compound (6.2) indicated above.

The "keto-paliperidone" (7.2) is then converted into paliperidone and/or to esters thereof under the same conditions as disclosed above.

While the use of a compound of formula (5.1) as a starting material is contemplated as a suitable use of the present invention, it is not limited thereto. The group G in formula (5) can be other than a leaving group, yet the above sequence of the reaction steps may be used, mutatis mutandis, for a conversion of compounds of the formula (5) to a compound of formula (1). When G in the formula (5) represents 4-(6-fluoro-1,2-benzisoxazol-3-yl)-1-piperidinyl group, the compound can be expressed as formula (5.2), which is also known as risperidone. Nonetheless, the steps of oximation, deoximation, and reduction transformation outlined above can be applied, under similar reaction conditions, to convert risperidone (5.2) into a paliperidone compound (10) as shown below. This represents the variant (D) of the process of the present invention and is illustrated in the following reaction scheme:

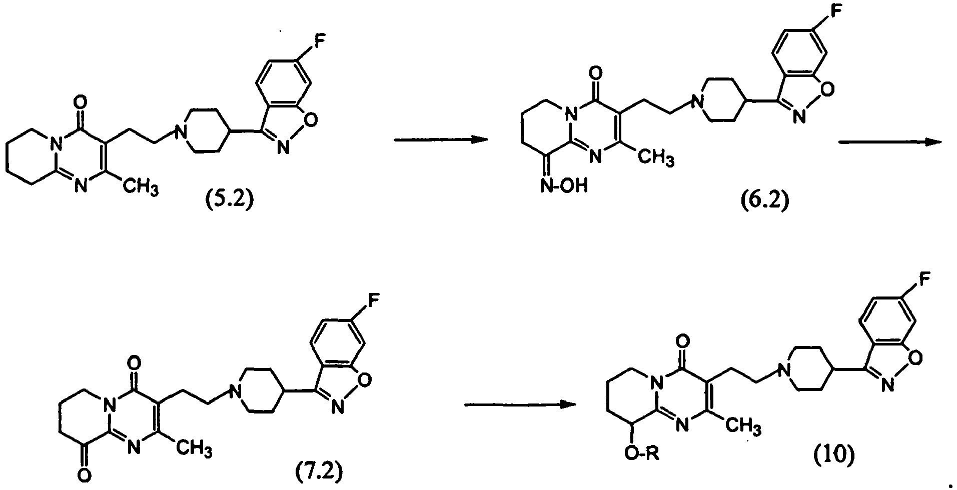

In a first step of the process, the risperidone compound (5.2) is converted into the compound (6.2) by an oximation reaction. The conditions of the oximation are essentially the same as the conditions of the conversion of the compound (5.1) into compound (6.1) disclosed above. The compound (6.2) may be isolated from the reaction mixture by conventional means, e.g., by precipitation from a reaction mixture or after an extraction with a suitable solvent, and is subjected to transformation reactions of deoximation and reduction outlined above, yielding the paliperidone compound (10) [R= H]. This compound may be converted into paliperidone esters (10) [R = C1-C20 alkyl], if desirable. The conversion from one group to the other can be carried out by methods and techniques known in the art and can afford further flexibility in the synthesis. As discussed above for the variants (B) and (C), the process can be monitored for the amount of the compound of formula (7.2) that is present.

In summary, the above process of the present invention starts from cheap starting materials, does not require using protective groups, runs under mild reaction conditions and proceeds with good yields and purity of the product. Accordingly, the whole process for making paliperidone and esters thereof can be more economical. In addition, it allows single enantiomers of paliperidone and esters thereof to be made by a simple process, which is advantageous from pharmaceutical point of view. The stereoselective reduction of keto-compounds of the general formula (7) (particularly (7.1) and (7.2)) according to the present invention is a useful process of making single enantiomers of paliperidone without a need of resolving enantiomers.

The invention will be further described with reference to the following nonlimiting examples.

### EXAMPLES

### Example 1 - Compound (6a)

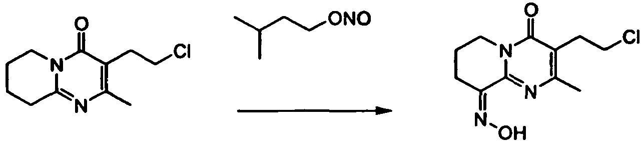

| | |
|---|---|
| 20.0 g | of 3-(2-chloroethyl)-6,7,8,9-tetrahydro-2-methyl-4H-pyrido[1,2-a] pyrimidin-4-one was suspended in |
| 20.0 ml | of n-butanol and the suspension was heated to 85 - 90°C. Then |
| 30.0 ml | of isoamyl nitrite was dropped during 1.5 h. |
| | Reaction mixture was diluted with |
| 50 ml | of ethyl acetate and the suspension was cooled to 20°C. Product was filtered off after 1 h of stirring and washed with |
| 2* 15 ml | of ethyl acetate. |

Yield: 19.39 g (86.0% of the theoretical yield), 98% purity (HPLC)

### Example 2 - Compound (1a)

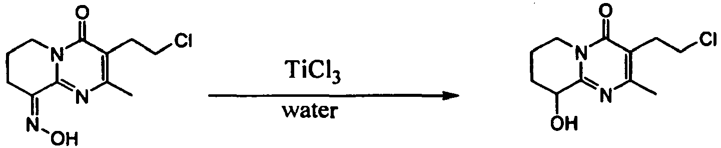

| | |
|---|---|
| 0.50 g | of the compound (6a) was suspended in |
| 9.8 ml | of 1M HCl under nitrogen and |
| 8.4 ml | of titanium(III) chloride were added. The solution was heated at 70°C for 30 minutes. |
| | The reaction mixture was diluted with |
| 20 ml | of water and the solution was alkalized with sodium bicarbonate. |
| | The suspension was extracted with |
| 4*25 ml | of DCM. The organic solution was dried with sodium sulphate and evaporated. |

**Yield**: 0.41 g (87% of theoretical yield), purity 96.8 %

### Example 3 - Compound (6.2)

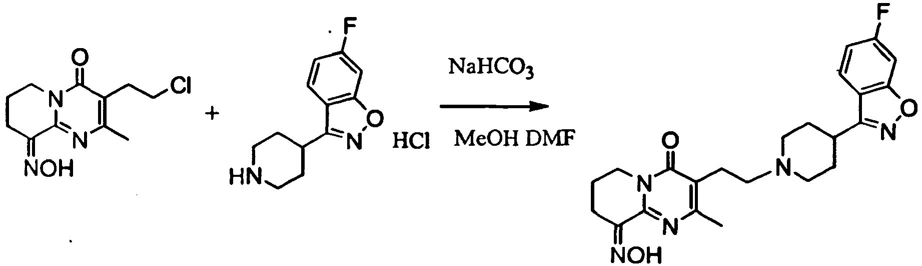

To the mixture of

| | |
|---|---|
| 11.24g | of compound (6a) in |
| 18.3 ml | of DMF and |
| 6.7 ml | of methanol, successively |
| 9.88 g | of 4-(6-fluoro-1,2-benzisoxazol-3-yl)piperidine.HCl and |
| 10.67 g | of sodium bicarbonate were added. The bright yellow suspension was heated and stirred at atmosphere N₂ for 3 hours at 70C, for 2 hours at 80C and for 3 hours at 85-90C. Then |
| 73 ml | of ethyl acetate was added into the reaction mixture at 80°C. Reaction mixture was cooled to 20°C.The suspension was stirred for 30 minutes. The crystals were filtered off and washed with |
| 2*18 ml | of ethyl acetate and |
| .2* 15 ml | of water. |

**Yield**:13.83 g (81.8% of the theoretical yield), purity 97.7%

### Example 4 - Synthesis of paliperidone

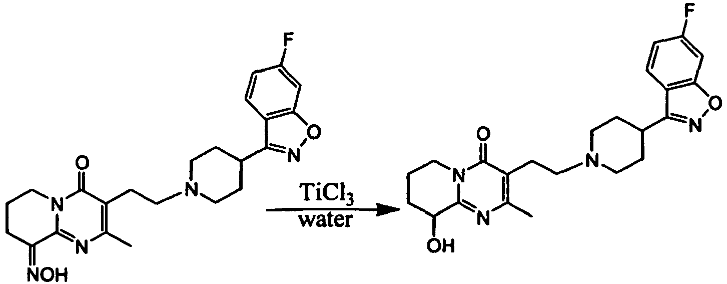

| | |
|---|---|
| 0.50 g | of the compound (6.2) was suspended in |
| 5.7 ml | of 1M HCl under nitrogen and |
| 4.9 ml | of titanium(III) chloride were heated at 70°C for 30 minutes. The reaction mixture was diluted with |
| 30 ml | of water and the solution was alkalized with sodium bicarbonate (cca 5 g). |

The mixture was extracted with

| | |
|---|---|
| 4*25 ml | of CHCl₃. The organic solution was dried with sodium sulphate and evaporated. |

The residue was: 0.42 g (86.50% of theoretical yield) of yellow oil. The yellow oil was diluted with

| | |
|---|---|
| 10 ml | of ethyl acetate. Crystals were filtered off and washed with |
| 2*2 ml | of ethyl acetate. |

**Yield**: 0.30 g(61.8% of theoretical yield) of paliperidone base. Purity: 96%.

## Claims

1. A compound of formula (6) or an acid addition salt thereof 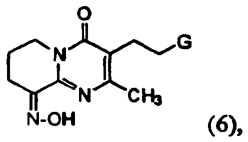 wherein G is selected from the group consisting of:
(i) a leaving group A selected from a halo group and a sulfonyloxy group, and
(ii) a group of the formula (20)

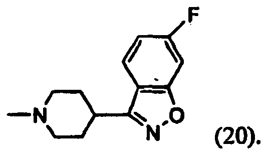

2. The compound according to claim 1, wherein G is a leaving group A selected from chloro, bromo, iodo, methanesulfonyloxy, trifluoromethanesulfonyloxy, benzenesulfonyloxy, 4-methylbenzenesulfonyloxy, and 4-methoxybenzenesulfonyloxy.

3. The compound according to claim 1, wherein G is chlorine.

4. The compound according to claim 1, wherein G is the group of the formula (20)

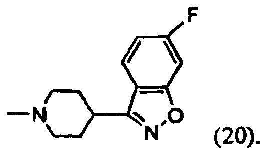

5. A process, which comprises:
a) reacting a compound of formula (5) or a salt thereof 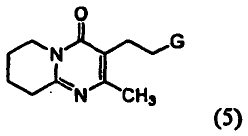 with a nitrite reagent to form a compound of formula (6) or a salt thereof 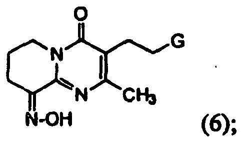
b) deoximating said compound of formula (6) or salt thereof to form a compound of formula (7) or a salt thereof 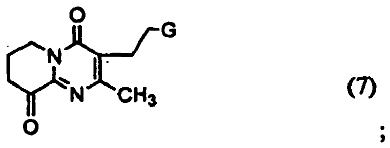 and
c) reducing said compound of formula (7) or salt thereof to form a compound of formula (1) or a salt thereof 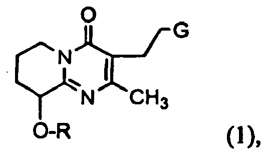 wherein G in each of the formulas (5), (6), (7), and (1) is selected from the group consisting of:
(i) a leaving group A selected from a halo group and a sulfonyloxy group, and
(ii) a group of the formula (20) 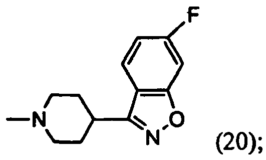 and
wherein R in the compound of formula (1) is hydrogen or a C1-C20 acyl group.

6. The process according to claim 5, wherein said leaving group A is a chloro, bromo, iodo, methanesulfonyloxy, trifluoromethanesulfonyloxy, benzenesulfonyloxy, 4-methylbenzenesulfonyloxy, or 4-methoxybenzenesulfonyloxy group.

7. The process according to claim 5, wherein when G in the compound of formula (5) is said leaving group A and said process further comprises converting the leaving group A on any one of the subsequent compounds of formulas (6), (7), and (1) into the group of the formula (20)

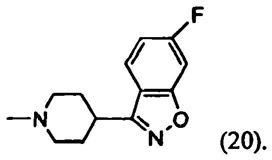

8. The process according to claim 5, wherein R in the compound of formula (1) is hydrogen and said process further comprises converting the hydrogen into a C1-C20 acyl group.

9. The process according to claim 5, wherein said deoximating step b) and said reducing step c) are performed as a single reaction step.

10. The process according to claim 5, wherein said compound of formula (6) formed in step a) is a racemate.

11. The process according to claim 5, wherein said compound of formula (1) formed in step c) is a racemate.

12. The process according to claim 5, wherein G in said compound of formula (1) is said leaving group A and said process further comprises reacting said compound of formula (1) or salt thereof with 4-(6-fluoro-1,2-benzisoxazol-3-yl)piperidine or a precursor thereof to covert said compound of formula (1) or salt thereof into a compound of formula (10) or a salt thereof 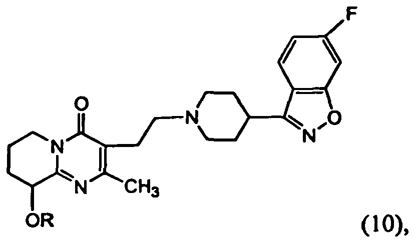 wherein R is hydrogen or a C1-C20 acyl group.

13. The process according to claim 5, wherein said compound of formula (5) is a compound of formula (5a) or a salt thereof, said compound of formula (6) is a compound of formula (6a) or a salt thereof, said compound of formula (7) is a compound of formula (7a) or a salt thereof, and said compound of formula (1) is a compound of formula (1a) or a salt thereof

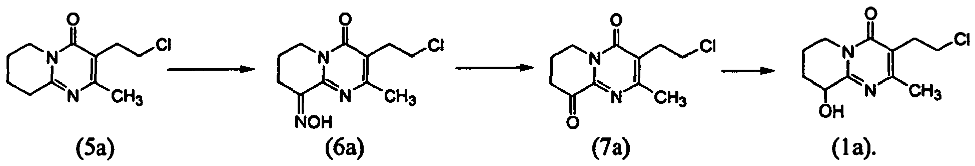

14. The process according to claim 13, which further comprises forming said compound of formula (5a) according to the following reaction scheme

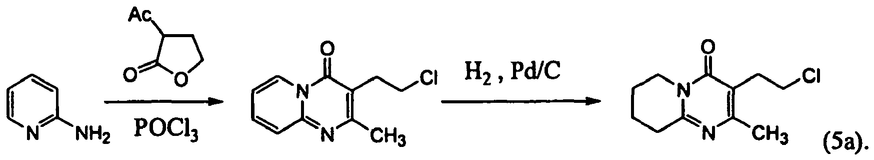

15. The process according to claim 14, which further comprises reacting said compound of formula (1a) or salt thereof with 4-(6-fluoro-1,2-benzisoxazol-3-yl)piperidine or a precursor thereof to covert said compound of formula (1a) or salt thereof into a compound of formula (10) or a salt thereof 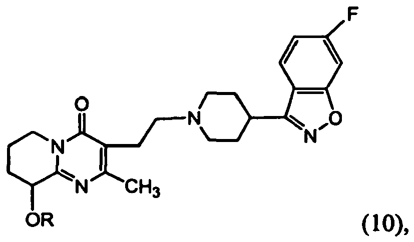 wherein R is hydrogen or a C1-C20 acyl group.

16. A process, which comprises:
a) reacting a compound of formula (5.1) or a salt thereof 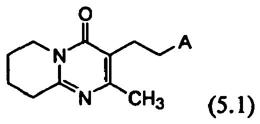 with a nitrite reagent to form a compound of formula (6.1) or a salt thereof 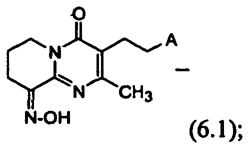
b) converting said compound of formula (6.1) or salt thereof into a compound of formula (7.2) or a salt thereof 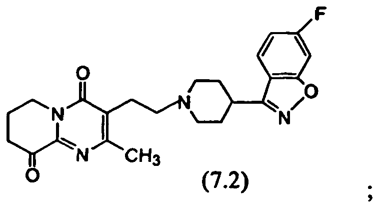 and
c) reducing said compound of formula (7.2) or salt thereof to form a compound of formula (10) or a salt thereof 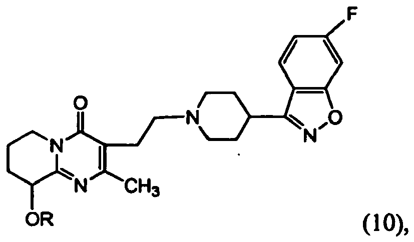 wherein said leaving group A in each of formulas (5.1) and (6.1) is selected from a halo group and a sulfonyloxy group, and
wherein R in formula (10) is hydrogen or a C1-C20 acyl group.

17. The process according to claim 16, wherein said converting of said compound of formula (6.1) into a compound of formula (7.2) comprises:
(i) reacting said compound of formula (6.1) or salt thereof with 4-(6-fluoro-1,2-benzisoxazol-3-yl)piperidine to form a compound of formula (6.2) or a salt thereof 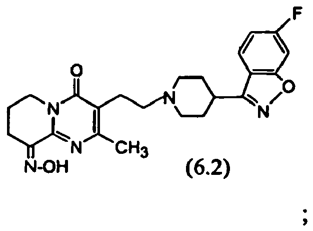 and
(ii) deoximating said compound of formula (6.2) or salt thereof to form said compound of formula (7.2) or salt thereof.

18. The process according to claim 16, wherein said converting of the compound of formula (6.1) into a compound of formula (7.2) comprises:
(i) deoximating said compound of formula (6.1) or salt thereof to form a compound of formula (7.1) or a salt thereof 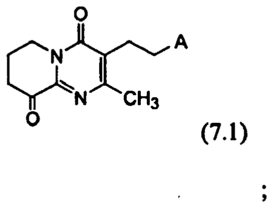 and
(ii) reacting said compound of formula (7.1) or salt thereof with 4-(6-fluoro-1,2-benzisoxazol-3-yl)piperidine to form said compound of formula (7.2) or salt thereof.

19. A process, which comprises:
a) reacting risperidone with a nitrite reagent to form a compound of formula (6.2) or a salt thereof 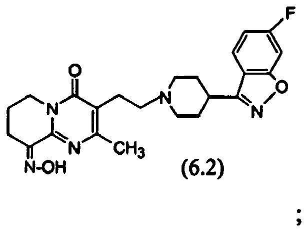
b) deoximating said compound of formula (6.2) or salt thereof into a compound of formula (7.2) or a salt thereof 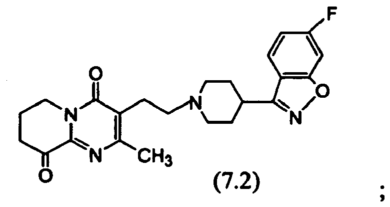 and
c) reducing said compound of formula (7) or salt thereof into a compound of formula (10) or a salt thereof 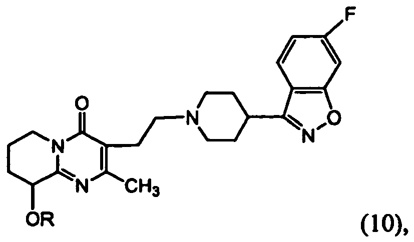 wherein R in the compound of formula (10) is hydrogen or a C1-C20 acyl group.

## Patentansprüche

1. Verbindung der Formel (6) oder eines Säureadditionssalzes derselben, 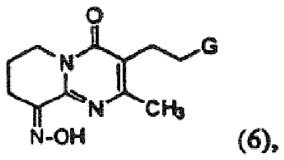 in der G ausgewählt ist aus der Gruppe bestehend aus:
(i) einer Abgangsgruppe A, die aus einer Halogengruppe und einer Sulfonyloxygruppe ausgewählt ist, und
(ii) einer Gruppe der Formel (20)

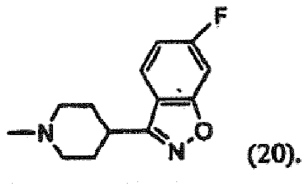

2. Verbindung nach Anspruch 1, bei der G eine Abgangsgruppe A ist, die ausgewählt ist aus Chlor, Brom, Iod, Methansulfonyloxy, Trifluormethansulfonyloxy, Benzolsulfonyloxy, 4-Methylbenzolsulfonyloxy und 4-Methoxybenzolsulfonyloxy.

3. Verbindung nach Anspruch 1, bei der G Chlor ist.

4. Verbindung nach Anspruch 1, bei der G die Gruppe der Formel (20) ist

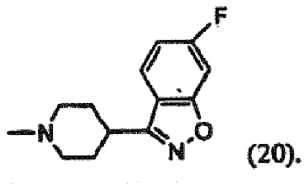

5. Verfahren, welches umfasst:
a) Umsetzen einer Verbindung der Formel (5) oder eines Salzes derselben 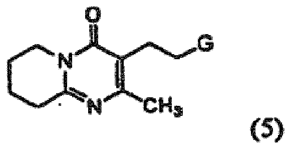 mit einem Nitrit-Reagens, um eine Verbindung der Formel (6) oder ein Salz derselben zu bilden 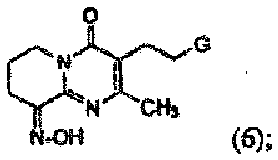
b) Deoximieren der Verbindung der Formel (6) oder des Salzes derselben,
um eine Verbindung der Formel (7) oder ein Salz derselben zu bilden 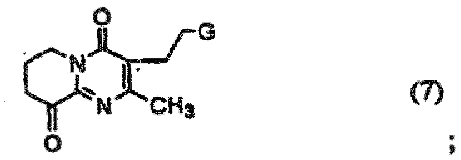 und
c) Reduzieren der Verbindung der Formel (7) oder des Salzes derselben, um eine Verbindung der Formel (1) oder ein Salz derselben zu bilden 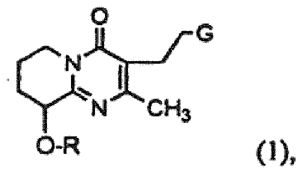 in der G in jeder der Formeln (5), (6), (7) und (1) ausgewählt ist aus der Gruppe bestehend aus:
(i) einer Abgangsgruppe A, die aus einer Halogengruppe und einer Sulfonyloxygruppe ausgewählt ist, und
(ii) einer Gruppe der Formel (20) 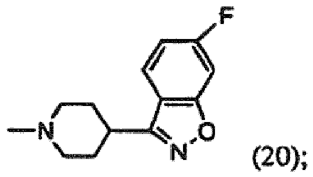
und
wobei R in der Verbindung der Formel (1) Wasserstoff oder eine C1-C20-Acylgruppe ist.

6. Verfahren nach Anspruch 5, bei dem die Abgangsgruppe A eine Chlor-, Brom-, Iod-, Methansulfonyloxy-, Trifluormethansulfonyloxy-, Benzolsulfonyloxy-, 4-Methylbenzolsulfonyloxy- oder 4-Methoxybenzolsulfonyloxy-Gruppe ist.

7. Verfahren nach Anspruch 5, bei dem, wenn G in der Verbindung der Formel (5) die Abgangsgruppe A ist, das Verfahren weiter die Umwandlung der Abgangsgruppe A an irgendeiner der anschließenden Verbindungen der Formeln (6), (7) und (1) in die Gruppe der Formel (20) 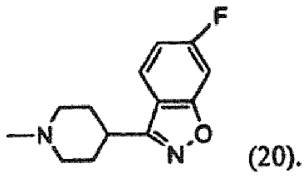 umfasst.

8. Verfahren nach Anspruch 5, bei dem R in der Verbindung der Formel (1) Wasserstoff ist und das Verfahren weiter die Überführung des Wasserstoffs in eine C1-C20-Acylgruppe umfasst.

9. Verfahren nach Anspruch 5, bei dem der Deoximierungsschritt b) und der Reduktionschritt c) als ein einziger Reaktionsschritt durchgeführt werden.

10. Verfahren nach Anspruch 5, wobei die Verbindung der Formel (6), die in Schritt a) gebildet wird, ein Racemat ist.

11. Verfahren nach Anspruch 5, wobei die Verbindung der Formel (1), die in Schritt c) gebildet wird, ein Racemat ist.

12. Verfahren nach Anspruch 5, bei dem G in der Verbindung der Formel (1) die Abgangsgruppe A ist und das Verfahren weiter die Umsetzung der Verbindung der Formel (1) oder des Salzes derselben mit 4-(6-Fluor-1,2-benzisoxazol-3-yl)piperidin oder einer Vorstufe desselben umfasst, um die Verbindung der Formel (1) oder das Salz derselben in eine Verbindung der Formel (10) oder ein Salz derselben 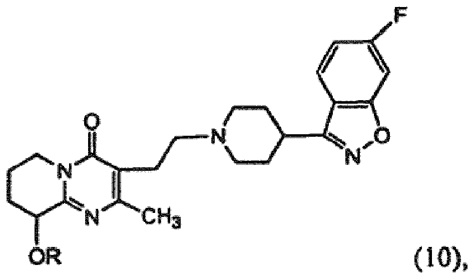 in der R Wasserstoff oder eine C1-C20-Acylgruppe ist, zu überführen.

13. Verfahren nach Anspruch 5, bei dem die Verbindung der Formel (5) eine Verbindung der Formel (5a) oder ein Salz derselben ist, die Verbindung der Formel (6) eine Verbindung der Formel (6a) oder ein Salz derselben ist, die Verbindung der Formel (7) eine Verbindung der Formel (7a) oder ein Salz derselben ist und die Verbindung der Formel (1) eine Verbindung der Formel (1a) oder ein Salz derselben ist

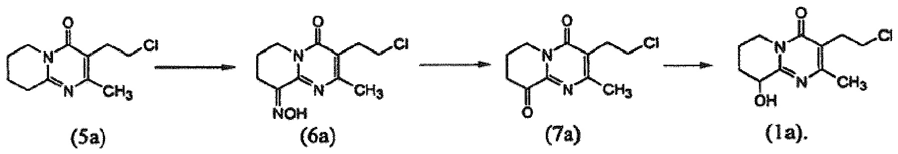

14. Verfahren nach Anspruch 13, das weiter die Bildung der Verbindung der Formel (5a) gemäß dem folgenden Reaktionsschema 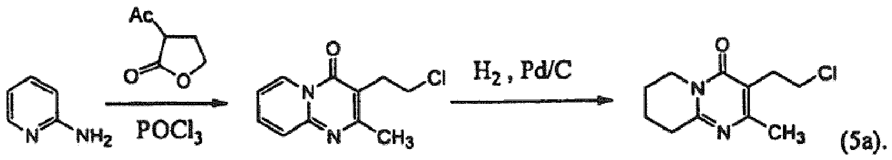 umfasst.

15. Verfahren nach Anspruch 14, das weiter die Umsetzung der Verbindung der Formel (1a) oder des Salzes derselben mit 4-(6-Fluor-1,2-benzisoxazol-3-yl)piperidin oder einer Vorstufe desselben umfasst, um die Verbindung der Formel (1 a) oder das Salz derselben in eine Verbindung der Formel (10) oder ein Salz derselben 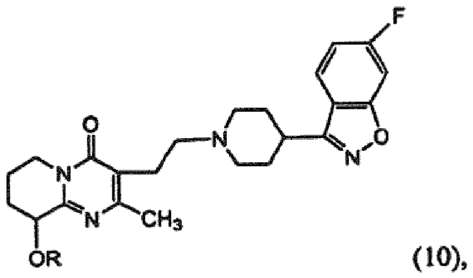 in der R Wasserstoff oder eine C1-C20-Acylgruppe ist, zu überführen.

16. Verfahren, welches umfasst
a) Umsetzen einer Verbindung der Formel (5.1) oder eines Salzes derselben 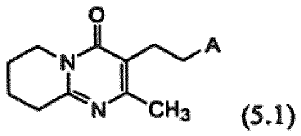 mit einem Nitrit-Reagens, um eine Verbindung der Formel (6.1) oder ein Salz derselben zu bilden, 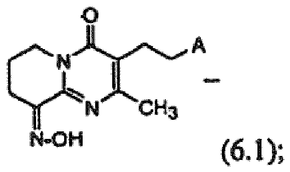
b) Überführen der Verbindung der Formel der Verbindung (6.1) oder des Salzes derselben in eine Verbindung der Formel (7.2) oder ein Salz derselben 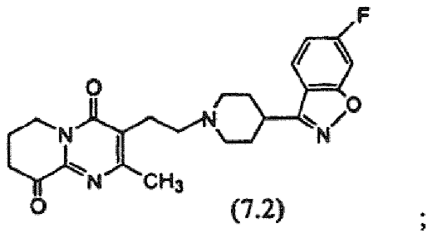 und
c) Reduzieren der Verbindung der Formel (7.2) oder des Salzes derselben, um eine Verbindung der Formel (10) oder ein Salz derselben zu bilden, 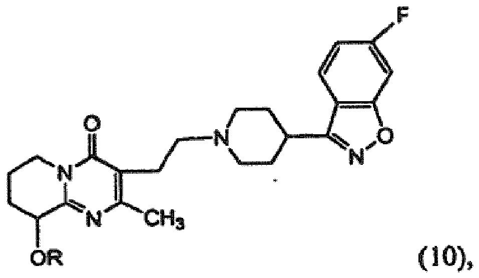 wobei die Abgangsgruppe A in jeder der Formeln (5.1) und (6.1) aus einer Halogengruppe und einer Sulfonyloxygruppe ausgewählt ist und
wobei R in der Formel (10) Wasserstoff oder eine C1-C20-Acylgruppe ist.

17. Verfahren nach Anspruch 16, bei dem die Überführung der Verbindung der Formel (6.1) in eine Verbindung der Formel (7.2) umfasst:
(i) Umsetzen der Verbindung der Formel (6.1) oder des Salzes derselben mit 4-(6-Fluor-1,2-benzisoxazol-3-yl)piperidin, um eine Verbindung der Formel (6.2) oder ein Salz derselben zu bilden 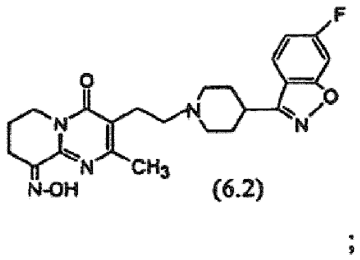 und
(ii) Deoximieren der Verbindung der Formel (6.2) oder des Salzes derselben, um die Verbindung der Formel (7.2) oder das Salz derselben zu bilden.

18. Verfahren nach Anspruch 16, bei dem die Überführung der Verbindung der Formel (6.1) in eine Verbindung der Formel (7.2) umfasst:
(i) Deoximieren der Verbindung der Formel (6.1) oder des Salzes derselben, um eine Verbindung der Formel (7.1) oder ein Salz derselben zu bilden, 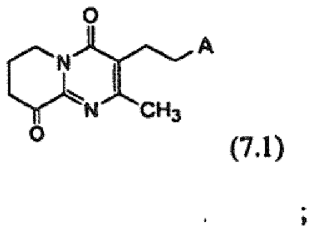 und
(ii) Umsetzen der Verbindung der Formel (7.1) oder des Salzes derselben mit 4-(6-Fluor-1,2-benzisoxazol-3-yl)piperidin, um die Verbindung der Formel (7.2) oder das Salz derselben zu bilden.

19. Verfahren, welches umfasst:
a) Umsetzen von Risperidon mit einem Nitrit-Reagens, um eine Verbindung der Formel (6.2) oder ein Salz derselben zu bilden 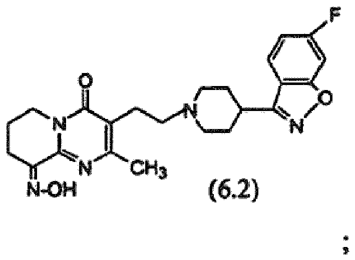
b) Deoximieren der Verbindung der Formel (6.2) oder des Salzes derselben zu einer Verbindung der Formel (7.2) oder einem Salz derselben 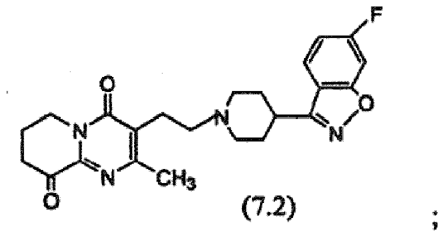 und
c) Reduzieren der Verbindung der Formel (7) oder des Salzes derselben zu einer Verbindung der Formel (10) oder einem Salz derselben 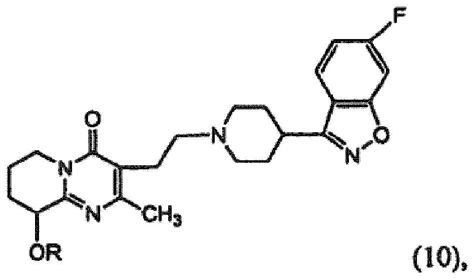 wobei R in der Verbindung der Formel (10) Wasserstoff oder eine C1-C20-Acylgruppe ist.

## Revendications

1. Composé de formule (6) ou sel d'addition d'acide de celui-ci 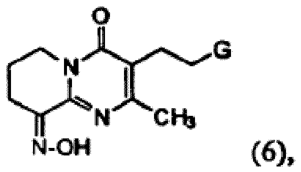 dans laquelle G est choisi dans le groupe constitué par :
(i) un groupe labile A choisi parmi un groupe halogéno et un groupe sulfonyloxy, et
(ii) un groupe de formule (20)

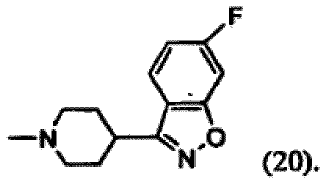

2. Composé selon la revendication 1, dans lequel G est un groupe labile A choisi parmi chloro, bromo, iodo, méthanesulfonyloxy, trifluorométhanesulfonyloxy, benzènesulfonyloxy, 4-méthylbenzène-sulfonyloxy, et 4-méthoxybenzènesulfonyloxy.

3. Composé selon la revendication 1, dans lequel G est un atome de chlore.

4. Composé selon la revendication 1, dans lequel G est le groupe de formule (20)

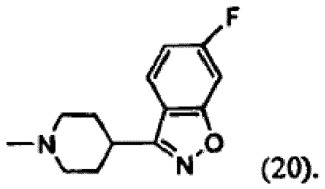

5. Procédé, qui comprend :
a) la réaction d'un composé de formule (5) ou d'un sel de celui-ci 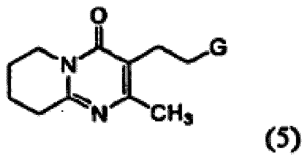 avec un réactif de nitrite pour former un composé de formule (6) ou un sel de celui-ci 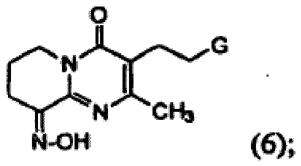
b) la désoximation dudit composé de formule (6) ou sel de celui-ci pour former un composé de formule (7) ou un sel de celui-ci 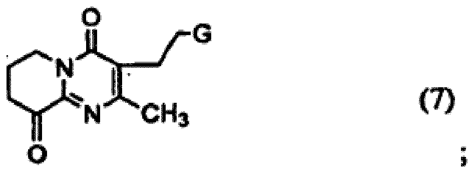 et
c) la réduction dudit composé de formule (7) ou sel de celui-ci pour former un composé de formule (1) ou un sel de celui-ci 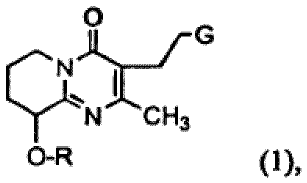 dans laquelle G dans chacune des formules (5), (6), (7), et (1) est choisi dans le groupe constitué par :
(i) un groupe labile A choisi parmi un groupe halogéno et un groupe sulfonyloxy, et
(ii) un groupe de formule (20) 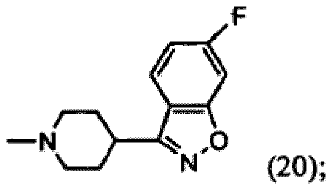 et
dans laquelle R dans le composé de formule (1) es un hydrogène ou un groupe acyle C1-C20.

6. Procédé selon la revendication 5, dans lequel ledit groupe labile A est un groupe chloro, bromo, iodo, méthanesulfonyloxy, trifluorométhanesulfonyloxy, benzènesulfonyloxy, 4-méthylbenzène-sulfonyloxy, ou 4-méthoxybenzènesulfonyloxy.

7. Procédé selon la revendication 5 dans lequel, quand G dans le composé de formule (5) est ledit groupe labile A, ledit procédé comprend, en outre, la conversion du groupe labile A sur l'un quelconque des composés ultérieurs de formules (6), (7), et (1) en groupe de formule (20)

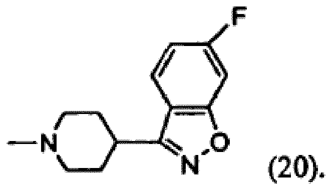

8. Procédé selon la revendication 5, dans lequel R dans le composé de formule (1) est un hydrogène et ledit procédé comprend, en outre, la conversion de l'hydrogène en un groupe acyle C1-C20.

9. Procédé selon la revendication 5, dans lequel ladite étape b) de désoximation et ladite étape c) de réduction sont mises en oeuvre comme une seule et même étape réactionnelle.

10. Procédé selon la revendication 5, dans lequel ledit composé de formule (6) formé dans l'étape a) est un racémate.

11. Procédé selon la revendication 5, dans lequel ledit composé de formule (1) formé dans l'étape c) est un racémate.

12. Procédé selon la revendication 5, dans lequel G dans ledit composé de formule (1) est ledit groupe labile A et ledit procédé comprend, en outre, la réaction dudit composé de formule (1) ou sel de celui-ci avec une 4-(6-fluoro-1,2-benzisoxazol-3-yl)pipéridine ou un précurseur de celle-ci pour convertir ledit composé de formule (1) ou sel de celui-ci en composé de formule (10) ou sel de celui-ci 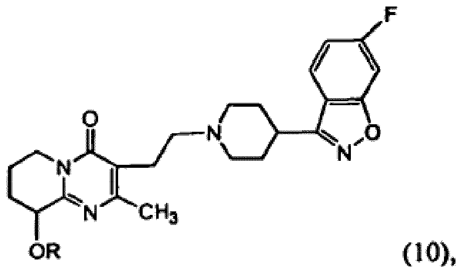 dans laquelle R est un hydrogène ou un groupe acyle C1-C20.

13. Procédé selon la revendication 5, dans lequel ledit composé de formule (5) est un composé de formule (5a) ou un sel de celui-ci, ledit composé de formule (6) est un composé de formule (6a) ou un sel de celui-ci, ledit composé de formule (7) est un composé de formule (7a) ou un sel de celui-ci, et ledit composé de formule (1) est un composé de formule (1a) ou un sel de celui-ci

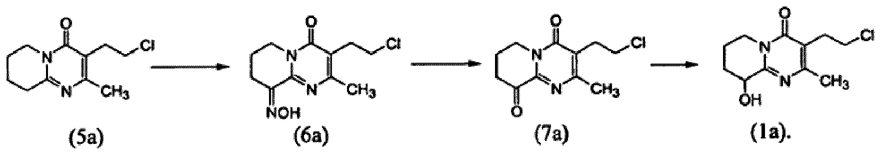

14. Procédé selon la revendication 13 qui comprend, en outre, la formation dudit composé de formule (5a) selon le schéma réactionnel qui suit

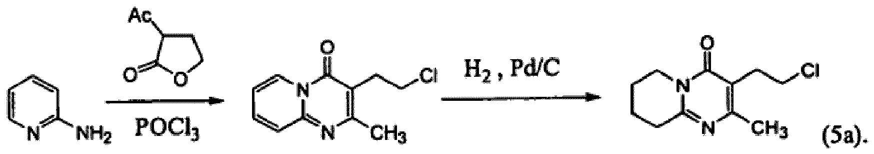

15. Procédé selon la revendication 14, qui comprend, en outre, la réaction dudit composé de formule (1a) ou sel de celui-ci avec une 4-(6-fluoro-1,2-benzisoxazol-3-yl)pipéridine ou un précurseur de celle-ci pour convertir ledit composé de formule (1a) ou sel de celui-ci en composé de formule (10) ou sel de celui-ci 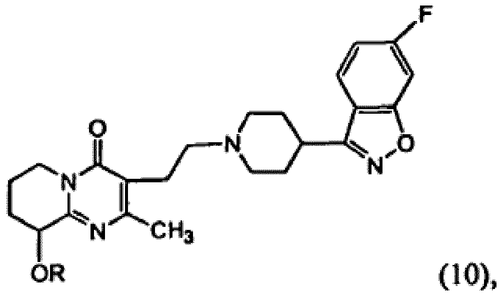 dans laquelle R est un hydrogène ou un groupe acyle C1-C20.

16. Procédé, qui comprend :
a) la réaction d'un composé de formule (5.1) ou d'un sel de celui-ci 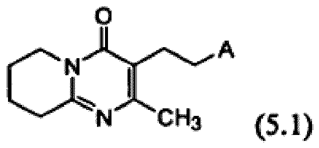 avec un réactif de nitrite pour former un composé de formule (6.1) ou un sel de celui-ci 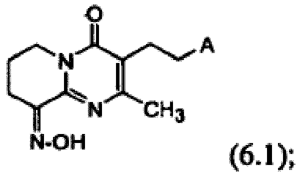
b) la conversion dudit composé de formule (6.1) ou sel de celui-ci en composé de formule (7.2) ou sel de celui-ci 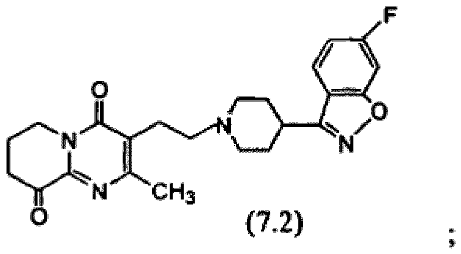 et
c) la réduction dudit composé de formule (7.2) ou sel de celui-ci pour former un composé de formule (10) ou un sel de celui-ci 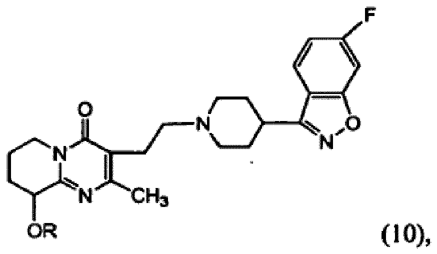 dans laquelle ledit groupe labile A dans chacune des formules (5.1) et (6.1) est choisi parmi un groupe halogéno et un groupe sulfonyloxy, et dans laquelle R dans la formule (10) est un hydrogène ou un groupe acyle C1-C20.

17. Procédé selon la revendication 16, dans lequel ladite conversion dudit composé de formule (6.1) en composé de formule (7.2) comprend :
(i) la réaction dudit composé de formule (6.1) ou sel de celui-ci avec une 4-(6-fluoro-1,2-benzisoxazol-3-yl)pipéridine pour former un composé de formule (6.2) ou un sel de celui-ci 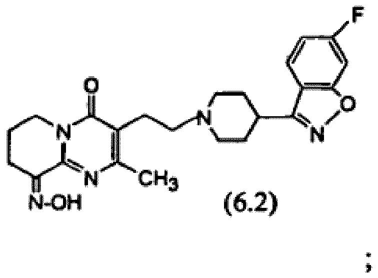 et
(ii) la désoximation dudit composé de formule (6.2) ou sel de celui-ci pour former ledit composé de formule (7.2) ou sel de celui-ci.

18. Procédé selon la revendication 16, dans lequel ladite conversion du composé de formule (6.1) en composé de formule (7.2) comprend :
(i) la désoximation dudit composé de formule (6.1) ou sel de celui-ci pour former un composé de formule (7.1) ou un sel de celui-ci 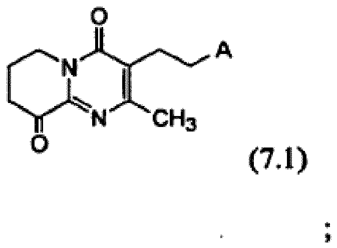 et
(ii) la réaction dudit composé de formule (7.1) ou sel de celui-ci avec une 4-(6-fluoro-1,2-benzisoxazol-3-yl)pipéridine pour former ledit composé de formule (7.2) ou sel de celui-ci.

19. Procédé, qui comprend :
a) la réaction d'une rispéridone avec un réactif de nitrite pour former un composé de formule (6.2) ou un sel de celui-ci 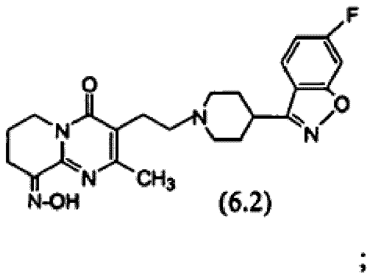
b) la désoximation dudit composé de formule (6.2) ou sel de celui-ci en composé de formule (7.2) ou sel de celui-ci 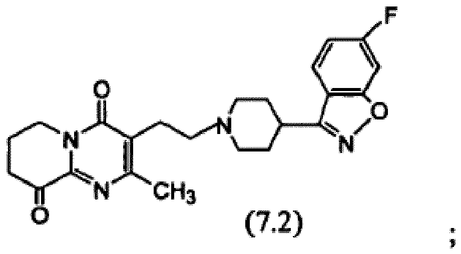 et
c) la réduction dudit composé de formule (7.2) ou sel de celui-ci
en composé de formule (10) ou sel de celui-ci 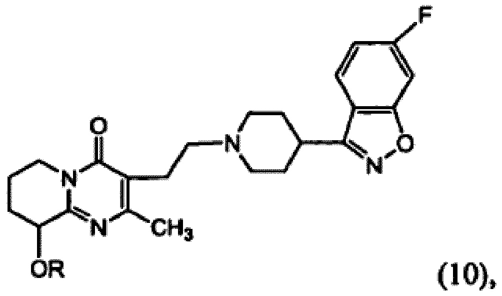 dans laquelle R dans le composé de formule (10) est un hydrogène ou un groupe acyle C1-C20.
